# EUROPÄISCHE PATENTSCHRIFT

(11) **EP 3 873 320 B1**
(45) Veröffentlichungstag und Bekanntmachung des Hinweises auf die Patenterteilung: **27.08.2025**
(21) Anmeldenummer: 19797362.1
(22) Anmeldetag: 25.10.2019
(51) Int. Cl.: A61B 1/005, A61B 1/00, A61B 1/01

(54) **ENDOSKOPSTEUERVORRICHTUNG UND ENDOSKOP MIT EINER ENDOSKOPSTEUERVORRICHTUNG**
ENDOSCOPE CONTROL DEVICE AND ENDOSCOPE WITH AN ENDOSCOPE CONTROL DEVICE
DISPOSITIF DE COMMANDE D'ENDOSCOPE ET ENDOSCOPE AVEC UN DISPOSITIF DE COMMANDE D'ENDOSCOPE

(30) Priorität: 29.10.2018 DE 102018126938
(43) Veröffentlichungstag der Anmeldung: 08.09.2021
(73) Patentinhaber: HOYA CORPORATION, Shinjuku-ku Tokyo 160-8347 (JP)
(72) Erfinder: DO, Anh Minh, 86316 Friedberg (DE)
(74) Vertreter: TBK
(86) Internationale Anmeldenummer: PCT/IB2019/001087
(87) Internationale Veröffentlichungsnummer: WO 2020/089685

(56) Entgegenhaltungen:
- JP-A- 2016 221 024
- US-A1- 2008 015 631
- US-A1- 2008 065 116
- US-A1- 2009 069 842
- US-A1- 2009 299 344

## Beschreibung

Die vorliegende Erfindung bezieht sich auf eine Endoskopsteuervorrichtung mit einem Steuerkörperhalter und einem joystickartigen Steuerelement zum Bewirken einer Auslenkbewegung. Die vorliegende Erfindung bezieht sich ausserdemauf ein Endoskop mit einer solchen Endoskopsteuervorrichtung.

In einem bekannten Endoskop ist an einer proximalen Seite eine Endoskopsteuervorrichtung vorgesehen, durch die eine Auslenkbewegung bewerkstelligt wird. Üblicherweise wird dadurch eine Auslenkbewegung eines an einer distalen Seite angeordneten ausschwenkenden Elementes verwirklicht.

Beispielsweise offenbart US 2012/0302832 A1 ein Endoskop mit einer Endoskopsteuervorrichtung, bei der ein Joystick um einen Schwenkpunkt geschwenkt wird. Vom Joystick erstreckt sich ein plattenartiger Arm, an dem beabstandet zum Joystick Zugseile verankert sind. Die Zugseile erstrecken sich von einem Steuerkörperhalter zu einem distalen Abschnitt des Endoskops und sind an einem auszuschwenkenden Element verankert. Der Joystick schwenkt relativ zum Steuerkörperhalter. Dadurch wird die Schwenkbewegung des an der distalen Seite angeordneten ausschwenkenden Elementes verwirklicht.

US 2008/065116 A1 zeigt eine Endoskopsteuervorrichtung mit den Merkmalen des Oberbegriffs von Anspruch 1.

JP 2016 221024 A offenbart eine Endoskopsteuervorrichtung mit einem Körper, der aus verbundenen Platten zwischen einer unteren Basis und einer oberen Platte besteht. Die verbundenen Platten sind mit der Basis in Kontakt. Die obere Platte hat einen kleineren Durchmesser als die Basis, die Basis hat einen größeren Durchmesser als die verbundenen Platten.

US 2009//069842 A1 und US 2009/299344 A1 zeigen jeweils einen Innenaufbau eines Instrumentengriffkörpers. Hierbei ist ein proximales biegbares Element zwischen einem distalen Rohrabschnitt und einem proximalen Rohrabschnitt eingebettet. Der distale Rohrabschnitt und der proximale Rohrabschnitt haben den gleichen Außendurchmesser wie das zwischen ihnen befindliche das proximale biegbare Element.

Dokument US 2008/015631 A1 offenbart eine Endoskopsteuervorrichtung gemäß dem Oberbegriff des Anspruchs 1.

In einem Endoskopkopf besteht stets Bedarf an einer Platzeinsparung, um die benötigten Komponenten auf engem Raum vorteilhaft unterbringen zu können.

Die Aufgabe der Erfindung ist es somit, eine verbesserte Endoskopsteuervorrichtung zu schaffen, bei der möglichst wenig Komponenten platzsparend angeordnet sind. Vorteilhafterweise soll die Endoskopsteuervorrichtung einfach aufgebaut sein und möglichst geringe Produktionskosten verursachen.

Diese Aufgabe ist durch eine Endoskopsteuervorrichtung mit den Merkmalen von Anspruch 1 gelöst.

Vorteilhafte Weiterbildungen sind Gegenstand der abhängigen Ansprüche.

Die Erfindung betrifft somit eine Endoskopsteuervorrichtung mit einem Steuerkörperhalter, einem joystickartigen Steuerelement zum Bewirken einer Auslenkbewegung und zumindest einem Steuerdraht, der durch den Steuerkörperhalter geführt ist und der die Auslenkbewegung des Steuerelements zu einem zu steuernden Element im Endoskop überträgt. Das Steuerelement erstreckt sich vom Steuerkörperhalter in eine proximale Richtung, und am Steuerelement ist beabstandet vom Steuerkörperhalter der zumindest eine Steuerdraht befestigt. Das Steuerelement weist ein elastisches Stabelement auf, das bei Betätigen des Steuerelements zum Bewirken einer Auslenkbewegung abknickbar ist. Das elastische Stabelement hat zentrisch entlang seiner Längserstreckung am Außenumfang sich in radialer Richtung nach außen erstreckende parallele Scheiben. Die in Längsrichtung des Stabelementes gesehen Zwischenräume zwischen den Scheiben sind in der Anzahl der Steuerdrähte vorgesehen. Die Scheiben besitzen am Außenumfangsrand Führungsöffnungen zum Führen eines Steuerdrahtes und die Führungsöffnungen für den gleichen Steuerdraht sind in Längsrichtung des Stabelementes ausgerichtet. Die Führungsöffnungen für verschiedene Steuerdrähte sind in Umfangsrichtung des Stabelementes zueinander versetzt. Die Scheiben sind zwischen einem distalen Rohrabschnitt und einem proximalen Rohrabschnitt des elastischen Stabelementes einstückig eingebettet. Die Scheiben haben einen größeren Außendurchmesser als der distale Rohrabschnitt und der proximale Rohrabschnitt.

Da die Auslenkbewegung durch ein bloßes Abknicken des elastischen Stabelements realisiert wird, kann die Endoskopsteuervorrichtung mit sehr wenigen Komponenten auf einfache Weise platzsparend und kostengünstig aufgebaut sein. Eine große Anzahl an zusätzlichen Bauteilen wird vermieden.

Das Steuerelement kann einen Betätigungsabschnitt an der zum Steuerkörperhalter entgegengesetzten Seite haben, wobei bei Betätigen des Steuerelements zum Spannen des zumindest einen Steuerdrahts das Stabelement geknickt wird.

Der Betätigungsabschnitt kann als ein Abdeckelement ausgebildet sein und das proximale Ende des Stabelementes bedecken, wobei zwischen Stabelement und Abdeckelement der zumindest eine Steuerdraht geklemmt ist.

Das Stabelement kann eine vordefinierte Sollknickstelle aufweisen.

Die Endoskopsteuervorrichtung kann des Weiteren zwei ineinandergreifende halbkugelartige Lagerschalen aufweisen, von denen die erste Lagerschale am Steuerkörperhalter abgestützt ist und die zweite Lagerschale am proximalen Ende des Stabelementes abgestützt ist, wobei die beiden Lagerschalen das elastische Stabelement umgeben. Die beiden Lagerschalen führen die Abknickbewegung des elastischen Stabelements. Somit kann das Abknicken des elastischen Stabelements stabil und gemäß der gewünschten Auslenkabsicht ausgeführt werden.

Im Bereich des Außenumfangsrandes sind benachbarte Scheiben durch Überbrückungsabschnitte verbunden, wobei jeder Überbrückungsabschnitt an benachbarten Scheiben so ausgebildet ist, dass Umfangsrandabschnitte der benachbarten Scheiben an der zum Überbrückungsabschnitt diametral entgegengesetzten Seite zueinander hin biegbar sind, und wobei die Überbrückungsabschnitte von Scheibe zu Scheibe in Umfangsrichtung versetzt angeordnet sind.

Da vier Steuerdrähte angewendet werden, können die vier Steuerdrähte zu zwei Paaren aus relativ zum Stabelement gegenüberliegenden Steuerdrähten zusammengefasst werden, deren Enden jeweils verbunden sind. Dadurch wird die Anzahl der Steuerdrähte minimal gehalten, wobei dennoch eine Auslenkbewegung in allen Richtungen möglich ist.

Das elastische Stabelement kann relativ zum Steuerkörperhalter verschiebbar sein. Alternativ kann das elastische Stabelement am Steuerkörperhalter fixiert angeordnet sein.

Das elastische Stabelement kann aus Kunststoff oder Metall hergestellt sein.

Die Erfindung betrifft ferner ein Endoskop mit einer solchen Endoskopsteuervorrichtung. Die Erfindung ist auf jede Art an Endoskop anwendbar, bei dem eine Auslenkbewegung durch ein Steuerelement gesteuert wird.

Die vorstehend erläuterten Aspekte der vorliegenden Erfindung können geeignet kombiniert werden.

Kurzbeschreibung der Zeichnung
Fig. 1 zeigt eine schematische Seitenschnittansicht einer Endoskopsteuervorrichtung eines ersten Ausführungsbeispiels der vorliegenden Erfindung.
Fig. 2 zeigt eine schematische perspektivische Ansicht der Endoskopsteuervorrichtung des ersten Ausführungsbeispiels.
Fig. 3 zeigt eine schematische perspektivische Ansicht eines Steuerelements mit einem Scheibenelement der Endoskopsteuervorrichtung des ersten Ausführungsbeispiels.
Fig. 4 zeigt eine schematische Seitenansicht des Steuerelements der Endoskopsteuervorrichtung des ersten Ausführungsbeispiels.
Fig. 5 zeigt eine weitere schematische Seitenansicht des Steuerelements der Endoskopsteuervorrichtung des ersten Ausführungsbeispiels, wobei insbesondere Überbrückungsabschnitte am Scheibenelement gezeigt sind.
Fig. 6 zeigt eine schematische perspektivische Ansicht eines elastischen Stabelements der Endoskopsteuervorrichtung des ersten Ausführungsbeispiels ohne Zugseile.
Fig. 7 zeigt eine schematische Seitenansicht des elastischen Stabelements der Endoskopsteuervorrichtung des ersten Ausführungsbeispiels.

Nachstehend ist die vorliegende Erfindung detailliert unter Bezugnahme auf die Zeichnungen anhand von Ausführungsbeispielen beschrieben.

### Erstes Ausführungsbeispiel

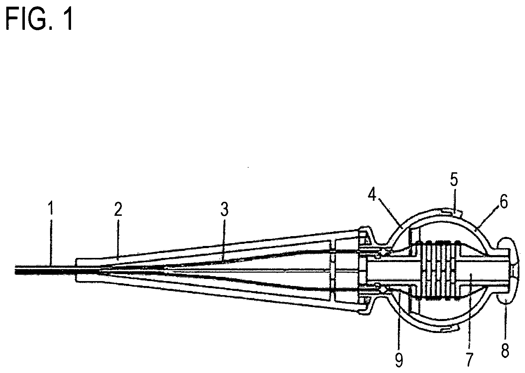

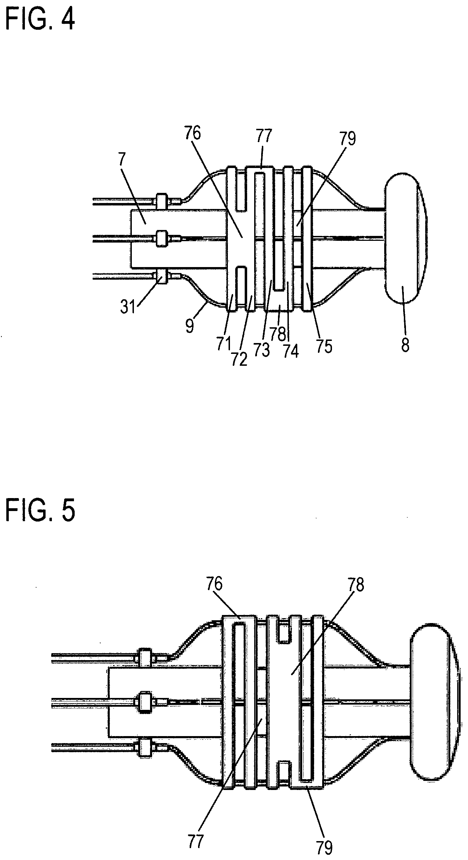

Nachstehend ist unter Bezugnahme auf die Figuren 1 bis 7 ein erstes Ausführungsbeispiel der vorliegenden Erfindung beschrieben.

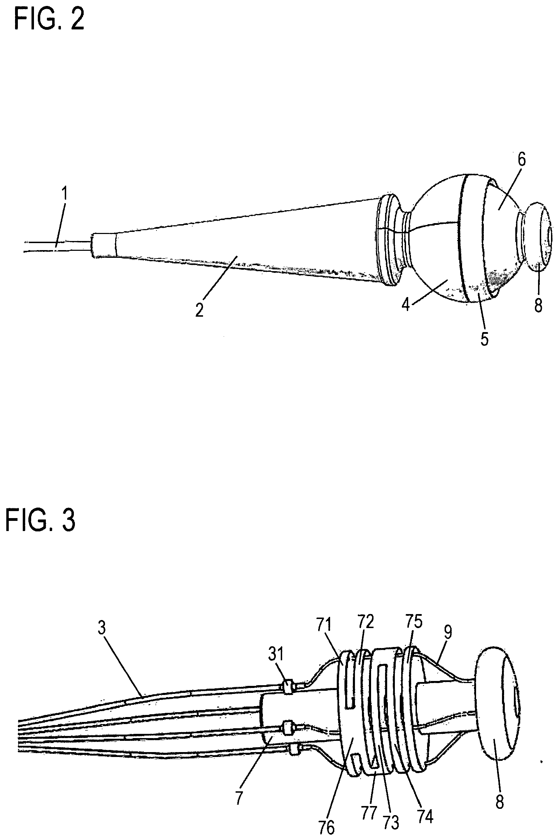

Fig. 1 zeigt eine schematische Seitenschnittansicht einer Endoskopsteuervorrichtung des ersten Ausführungsbeispiels. Fig. 2 zeigt eine schematische perspektivische Ansicht dieser Endoskopsteuervorrichtung.

Die Endoskopsteuervorrichtung bildet einen proximalen Abschnitt eines Endoskops. Das Endoskop hat einen elastischen Einführschlauch 1, der sich distal von einem Kontrollkörpergehäuse 2 erstreckt. Der Einführschlauch 1 wird in einen Hohlraum eines Patienten eingeführt. Der Einführschlauch 1 besitzt an der (nicht gezeigten) distalen Seite einen biegbaren Abschnitt, das heißt einen sogenannten Deflectingabschnitt, der relativ zum proximalen Teil des Einführschlauches 1 geschwenkt werden kann. Die Schwenkbewegung des Deflectingabschnittes wird durch Schwenken eines nachstehend erläuterten Steuerelementes gesteuert, wobei die Bewegung des Deflectingabschnittes genau der Bewegung des Steuerelementes folgt.

Zum Zwecke der Übertragung der Schwenkbewegung vom Steuerelement zum Deflectingabschnitt verlaufen vier Steuerdrähte 9 als Zugseile (Zugdrähte) vom Kontrollkörpergehäuse 2 im Einführschlauch 1 zum Deflectingabschnitt. Im Deflectingabschnitt sind die Steuerdrähte 9 in bekannter Weise am Umfang versetzt unter gleichen Abständen verankert. Daher sind die Verankerungsorte der Steuerdrähte 9 am Deflectingabschnitt um 90 Grad jeweils zueinander versetzt. Durch Ziehen eines Steuerdrahtes 9 wird der Deflectingabschnitt zu der Seite verschwenkt, an der sich der Verankerungsort des gezogenen Steuerdrahtes 9 befindet. In bekannter Weise sind die Steuerdrähte 9 in jeweiligen flexiblen Führungsfedern 3 im Einführschlauch 1 geführt. Die Führungsfedern 3 erstrecken sich vom proximalen Ende des Deflectingabschnittes im Einführschlauch 1 in proximaler Richtung bis in das Kontrollkörpergehäuse 2 hinein. Die jeweilige Führungsfeder 3 wirkt für den ihr zugewiesenen Steuerdraht 9 als Bowdenzugmantel.

Das Kontrollkörpergehäuse 2 ist aus Kunststoff hergestellt. Das Kontrollkörpergehäuse 2 ist in der Form eines Zylinders aufgebaut, dessen Durchmesser zur proximalen Seite allmählich zunimmt. Das Kontrollkörpergehäuse 2 hat eine Mittelachse. An der distalen Seite des Kontrollkörpergehäuses 2 erstreckt sich der Einführschlauch 1 in die distale Richtung. Der Einführschlauch 1 sitzt im Kontrollkörpergehäuse 2. Der Einführschlauch 1 bewegt sich nicht relativ zum Kontrollkörpergehäuse 2. Die Form des Kontrollkörpergehäuses 2 ist nicht beschränkt. Andere Formen können angewendet werden.

Das Kontrollkörpergehäuse 2 weist an seiner proximalen Seite einen Anschluß auf, an dem eine erste Lagerschale 4 fest angebracht ist. Die erste Lagerschale 4 bildet ein halbkugelförmiges Lager in der Form einer zur proximalen Seite offenen Halbkugel.

Genauer gesagt ist, wie in Fig. 2 gezeigt, die erste Lagerschale 4 in der Form eines Eierbechers aufgebaut. Die Seite des Eierbecherständers bildet die distale Seite der ersten Lagerschale 4 und ist am Kontrollkörpergehäuse 2 befestigt. Die proximale Seite bildet einen Lagerschalenkörper der ersten Lagerschale 4. Die erste Lagerschale 4 kann so hergestellt sein, dass zwei symmetrische Eierbecherhälften zusammengeklebt sind. Die Herstellung ist aber nicht darauf beschränkt. An der distalen Seite der ersten Lagerschale 4 ist ein Ringelement 5 angeordnet. Außerdem ist die erste Lagerschale 4 an der distalen Innenumfangsseite in einer zylindrischen Form gleichbleibenden Durchmessers ausgebildet.

Die erste Lagerschale 4 und das Ringelement 5 halten eine zweite Lagerschale 6. Die zweite Lagerschale 6 bildet einen halbkugelförmigen zu lagernden Körper in der Form einer zur distalen Seite offenen Halbkugel. Dabei wirken die erste Lagerschale 4 als erstes Gehäuseelement und das Ringelement 5 als zweites Gehäuseelement zum Unterbringen der zweiten Lagerschale 6.

Die erste Lagerschale 4 und das Ringelement 5 sind zumindest an ihrer Innenfläche kugelförmig. Die zweite Lagerschale 6 ist zumindest an ihrer Außenfläche kugelförmig. Die Kugelform der zweiten Lagerschale 6 und die Kugelform des Lagerschalenkörpers der ersten Lagerschale 4 und des Ringelemente 5 sind so gewählt, dass die zweite Lagerschale 6 sich in der ersten Lagerschale 4 und im Ringelement 5 relativ zur ersten Lagerschale 4 und zum Ringelement 5 bewegen kann. Dabei sitzt die distale Seite der zweiten Lagerschale 6 in der proximalen Seite d.h. im Lagerschalenkörper der ersten Lagerschale 4.

Das Ringelement 5 verhindert, dass die zweite Lagerschale 6 aus der ersten Lagerschale 4 in die distale Richtung entweicht.

Die erste Lagerschale 4, das Ringelement 5 und die zweite Lagerschale 6 sind aus Kunststoff hergestellt.

An der proximalen Seite der zweiten Lagerschale 6 geht die sich verjüngende Kugelform in eine zylindrische Form gleichbleibenden Durchmessers über. Die zylindrische Form gleichbleibenden Durchmessers der zweiten Lagerschale 6 erstreckt sich in die proximale Richtung.

Der Durchmesser des an der proximalen Seite der zweiten Lagerschale 6 ausgebildeten Zylinderabschnittes ist gleich dem Durchmesser des an der distalen Seite der ersten Lagerschale 4 am Innenumfang ausgebildeten Zylinderabschnittes.

Zwischen dem an der distalen Seite der ersten Lagerschale 4 am Innenumfang ausgebildeten Zylinderabschnitt und dem an der proximalen Seite der zweiten Lagerschale 6 ausgebildeten Zylinderabschnitt erstreckt sich ein Stabelement 7. Das Stabelement 7 wirkt als Steuerelement oder Steuerkörper im Sinne der Erfindung.

Das Kontrollkörpergehäuse 2 wirkt somit als Steuerkörperhalter.

Das Stabelement 7 ist ein rohrartiges Element. Das Stabelement 7 ist aus einem elastischen Material wie z.B. Kunststoff oder Metall hergestellt. Das Stabelement 7 erstreckt sich auf der verlängerten Mittelachse des Kontrollkörpergehäuses 2. Das Stabelement 7 hat einen distalen Rohrabschnitt 7A an der distalen Seite und einen proximalen Rohrabschnitt 7B an der proximalen Seite.

Die distale Seite des Stabelementes 7 wird von einem Innenumfangsabschnitt (des Eierbecherständers) der ersten Lagerschale 4 gehalten. Der Innenumfangsabschnitt der ersten Lagerschale 4 verhindert, dass sich der distale Rohrabschnitt 7A radial bewegen kann. Der Innenumfangsabschnitt der ersten Lagerschale 4 verhindert außerdem, dass sich der distale Rohrabschnitt 7A in Längsrichtung bewegen kann.

Am Außenumfang des distalen Rohrabschnittes 7A sind für die vier Führungsfedern 3 jeweilige Führungsfederhalter 31 feststehend angeordnet. Somit sind vier jeweilige Führungsfederhalter 31 vorgesehen.

Der Führungsfederhalter 31 wirkt als Zuggegenhalter. Der Führungsfederhalter 31 nimmt das proximale Ende der Führungsfeder 3 auf und führt die aus der Führungsfeder 3 resultierende Druckkraft in das Stabelement 7 ab. An der proximalen Seite des Führungsfederhalters 31 tritt daher der Steuerdraht 9 aus seiner Führungsfeder 3 und erstreckt sich in proximaler Richtung.

Die jeweiligen Führungsfederhalter 31 sind zwischen dem Innenumfangsabschnitt der ersten Lagerschale 4 und dem distalen Rohrabschnitt 7A so angeordnet, dass keine Relativverschiebung zwischen ihnen möglich ist.

Die proximale Seite des Stabelementes 7 wird von einem Innenumfangsabschnitt der zweiten Lagerschale 6 gehalten. Anders ausgedrückt verhindert der Innenumfangsabschnitt der zweiten Lagerschale 6, dass sich der proximale Rohrabschnitt 7B radial bewegen kann. Ferner sitzt an der proximalen Seite des proximalen Rohrabschnittes 7B und der zweiten Lagerschale 6 ein Abdeckelement 8, das ebenfalls ein Teil des Steuerelementes ist. Das Abdeckelement 8 bedeckt die proximale Seite des proximalen Rohrabschnittes 7B und der zweiten Lagerschale 6. Das Abdeckelement 8 verhindert dass sich der proximale Rohrabschnitt 7B in Längsrichtung relativ zum Innenumfangsabschnitt der zweiten Lagerschale 6 bewegen kann.

Somit wird eine Relativbewegung des proximalen Rohrabschnittes 7B zum distalen Rohrabschnitt 7A durch die erste Lagerschale 4 und die zweite Lagerschale 6 geführt. Die zweite Lagerschale 6 und das Abdeckelement 8 bilden einen Joystickkopf.

Die proximalen Enden der Steuerdrähte 9 sind dabei zwischen dem Innenumfangsabschnitt der zweiten Lagerschale 6 und dem proximalen Rohrabschnitt 7B geklemmt. Am proximalen Ende des proximalen Rohrabschnittes 7B sind die Steuerdrähte 9 nach innen gebogen und werden durch das Abdeckelement 8 geklemmt. Somit sorgt das Abdeckelement 8 dafür, dass eine Relativbewegung der Steuerdrähte 9 zum proximalen Endabschnitt des proximalen Rohrabschnittes 7B verhindert ist.

Der Verlauf der Steuerdrähte 9 ist hierbei derart, dass stets zwei in bezug auf den Durchmesser des proximalen Rohrabschnittes 7B gegenüberliegende Steuerdrähte 9 ein Paar bilden. Unter Betrachtung von Fig. 3 bilden somit ein vorderer Steuerdraht 9 und ein hinterer Steuerdraht 9 ein Paar, und bilden ein oberer Steuerdraht 9 und ein unterer Steuerdraht 9 ein Paar. An der distalen Seite sind am Deflectingabschnitt die ein Paar bildenden Steuerdrähte 9 verbunden. An der proximalen Seite sind die ein Paar bildenden Steuerdrähte 9 (z.B. durch Verknoten, Verschweißen etc.) verbunden. Der proximale Endabschnitt jedes Steuerdrahtes 9 geht somit in den proximalen Endabschnitt des zu ihm zugehörigen Steuerdrahtes 9 über, mit dem er ein Paar bildet. Somit werden im Ausführungsbeispiel streng genommen zwei Paare an Steuerdrähten 9 verwendet.

Zwischen dem distalen Rohrabschnitt 7A und dem proximalen Rohrabschnitt 7B hat das Stabelement 7 ein nachstehend beschriebenes einstückiges Scheibenelement.

Das Scheibenelement ist zwischen dem distalen Rohrabschnitt 7A und dem proximalen Rohrabschnitt 7B einstückig eingebettet.

Das Scheibenelement ist als ein zylindrischer Körper aufgebaut, der einen größeren Aussendurchmesser als der distale Rohrabschnitt 7A und der proximale Rohrabschnitt 7B hat. Das Scheibenelement ist aus in axialer Richtung gesehen fünf aufeinanderfolgenden Scheiben 71, 72, 73, 74, 75 gleicher Dicke aufgebaut. In Längsrichtung sind die aufeinanderfolgenden Scheiben 71, 72, 73, 74, 75 zueinander gleich beabstandet. Somit haben die Scheiben 71, 72, 73, 74, 75 Zwischenräume mit jeweils gleichem Längsrichtungsmaß. Jeweils zwei benachbarte Scheiben sind am Außenumfang durch einen Überbrückungsabschnitt 76, 77, 78, 79 verbunden. Von Scheibe zu Scheibe ist der Überbrückungsabschnitt in Umfangsrichtung um einen Viertel des Gesamtumfangs versetzt angeordnet. Die Scheiben 71, 72, 73, 74, 75 sind zueinander parallel.

Genauer gesagt sind die erste Scheibe 71 und die zweite Scheibe 72 am Außenumfang durch einen ersten Überbrückungsabschnitt 76 verbunden. Die zweite Scheibe 72 und die dritte Scheibe 73 sind am Außenumfang durch einen zweiten Überbrückungsabschnitt 77 verbunden. Die dritte Scheibe 73 und die vierte Scheibe 74 sind am Außenumfang durch einen dritten Überbrückungsabschnitt 78 verbunden. Die vierte Scheibe 74 und die fünfte Scheibe 75 sind am Außenumfang durch einen vierten Überbrückungsabschnitt 79 verbunden.

Der erste Überbrückungsabschnitt 76 ist zum zweiten Überbrückungsabschnitt 77 in Umfangsrichtung des Scheibenelements versetzt angeordnet. Der zweite Überbrückungsabschnitt 77 ist zum dritten Überbrückungsabschnitt 78 in Umfangsrichtung des Scheibenelements versetzt angeordnet. Der dritte Überbrückungsabschnitt 78 ist zum zweiten Überbrückungsabschnitt 77 in Umfangsrichtung des Scheibenelements versetzt angeordnet. Der vierte Überbrückungsabschnitt 79 ist zum dritten Überbrückungsabschnitt 78 in Umfangsrichtung des Scheibenelements versetzt angeordnet.

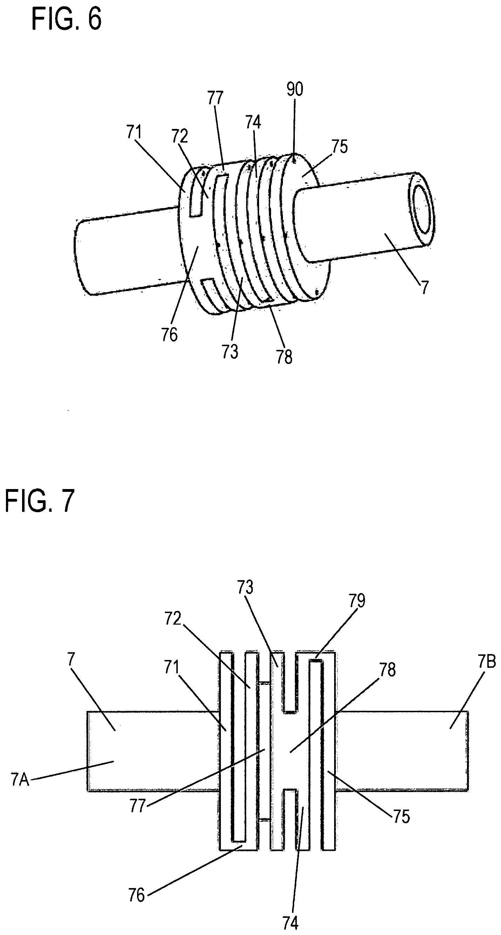

Das Scheibenelement wird z.B. so hergestellt, dass die Zwischenräume zwischen den Scheiben 71, 72, 73, 74, 75 z.B. per Laser so eingeschnitten werden, dass nur der geringfügige Überbrückungsabschnitt 76, 77, 78, 79 am Rand bestehen bleibt, siehe Figur 7.

Genauer gesagt wird ein Zwischenraum zwischen den Scheiben 71 und 72 per Laser so eingeschnitten, dass ein geringfügiger Überbrückungsabschnitt 76 am Rand bestehen bleibt. Das Scheibenelement wird danach um 90 Grad gedreht und der nächste Zwischenraum zwischen den Scheiben 72 und 73 wird so geschnitten, dass ein geringfügiger Überbrückungsabschnitt 77 am Rand bestehen bleibt. Das Scheibenelement wird danach um 180 Grad weitergedreht und der nächste Zwischenraum zwischen den Scheiben 73 und 74 wird so eingeschnitten, dass ein geringfügiger Überbrückungsabschnitt 78 am Rand bestehen bleibt. Das Scheibenelement wird danach um 90 Grad weitergedreht und der nächste Zwischenraum zwischen den Scheiben 74 und 75 wird so eingeschnitten, dass ein geringfügiger Überbrückungsabschnitt 79 am Rand bestehen bleibt.

Somit ist der proximale Rohrabschnitt 7B relativ zum distalen Rohrabschnitt 7A abknickbar, indem der proximale Rohrabschnitt 7B in die Richtung gebogen wird, die zu einem jeweiligen Überbrückungsabschnitt 76, 77, 78, 79 diametral entgegengesetzt ist. Z.B. weist in Fig. 3 der Überbrückungsabschnitt 76 zum Betrachter. Indem der proximale Rohrabschnitt 7B in der Darstellung von Figur 3 vom Betrachter weg gedrückt wird, knickt der proximal vom Überbrückungsabschnitt 76 gelegene Teil des Stabelementes 7 relativ zum distalen Rohrabschnitt 7A, wobei der Überbrückungsabschnitt 76 als Drehbereich (Drehpunkt) dient, bis der zum Überbrückungsabschnitt 76 entgegengesetzte Aussenumfangsabschnitt der ersten Scheibe 71 an dem zum Überbrückungsabschnitt 76 entgegengesetzten Aussenumfangsabschnitt der zweiten Scheibe 72 anliegt.

In ähnlicher Weise kann der proximale Rohrabschnitt 7B relativ zum distalen Rohrabschnitt 7A abgeknickt werden, indem der proximale Rohrabschnitt 7B seitlich vom Überbrückungsabschnitt 77, 78 oder 79 jeweils so gedrückt wird, dass der Überbrückungsabschnitt 77, 78 oder 79 jeweils als Drehpunkt dient.

Im Scheibenelement sind vier in Längsrichtung des Scheibenelements durchgehende Öffnungen 90 so vorgesehen, dass sie durch jede Scheibe 71, 72, 73, 74, 75 laufen, siehe Figur 6. Diese Öffnungen 90 sind Führungsöffnungen für die jeweiligen Steuerdrähte 9. Die vier in Längsrichtung des Scheibenelements durchgehende Öffnungen 90 sind ebenfalls zueinander z.B. um einen Viertel des Gesamtumfangs des Scheibenelements versetzt angeordnet.

Somit hat jeder Überbrückungsabschnitt 76, 77, 78, 79 einen Abschnitt, durch den sich eine Öffnung 90 erstreckt, siehe Figur 3. Aus Figur 3 ist ersichtlich, dass der erste Überbrückungsabschnitt 76 eine Öffnung 90 für den vorderen Steuerdraht 9 besitzt. Der zweite Überbrückungsabschnitt 77 hat eine Öffnung 90 für den unteren Steuerdraht 9 in Figur 3. Der dritte Überbrückungsabschnitt 78 hat eine Öffnung 90 für den oberen Steuerdraht 9. Der vierte Überbrückungsabschnitt 79 hat eine Öffnung 90 für den hinteren Steuerdraht 9.

Das Scheibenelement sorgt somit dafür, dass bei Betätigung (Biegen) des Stabelementes 7 für jeden Steuerdraht 9 eine vordefinierte Sollknickstelle im Stabelement 7 vorgesehen ist.

Wenn z.B. der proximale Rohrabschnitt 7B in der Darstellung von Figur 3 vom Betrachter weg gedrückt wird, knickt der proximale Rohrabschnitt 7B relativ zum distalen Rohrabschnitt 7A, wobei der Überbrückungsabschnitt 76 als Drehbereich (Drehpunkt) dient. Somit wird der vordere Steuerdraht 9 gespannt (gezogen) und der hintere Steuerdraht 9 wird entspannt. Das Paar aus dem vorderen Steuerdraht 9 und dem hinteren Steuerdraht 9 sorgen somit für eine Auslenkbewegung an der distalen Seite des Endoskops, die der Drückbewegung am proximalen Rohrabschnitt 7B entspricht.

Diese Drückbewegung am proximalen Rohrabschnitt 7B erfolgt im Ausführungsbeispiel am Abdeckelement 8 und wird durch die Lagerschalen 4 und 6 geführt.

Im Ausführungsbeispiel wird dadurch eine Auslenkbewegung am distalen Deflectingabschnitt durch einen einfach und kostengünstig herstellbaren Aufbau aus wenigen Einzelteilen ermöglicht.

### Alternativen und weitere Ausführungsbeispiele

Die erläuterten Ausführungsbeispiele können geeignet kombiniert werden, sofern sich dadurch kein technischer Widerspruch ergibt.

Im Ausführungsbeispiel wird die distale Seite des Stabelementes 7 vom Innenumfangsabschnitt der ersten Lagerschale 4 gehalten. D.h. der Innenumfangsabschnitt der ersten Lagerschale 4 verhindert, dass sich der distale Rohrabschnitt 7A radial und in Längsrichtung bewegen kann. In einer Alternative wird die distale Seite des Stabelementes 7 vom Innenumfangsabschnitt der ersten Lagerschale 4 so gehalten, dass sich der distale Rohrabschnitt 7A radial nicht bewegen kann aber sich in Längsrichtung bewegen kann. Somit ist eine geringfügige Verschiebung des Stabelementes 7 relativ zum Innenumfangsabschnitt der ersten Lagerschale 4 in Längsrichtung des Stabelementes 7 möglich. In diesem Beispiel ist das elastische Stabelement 7 relativ zum Steuerkörperhalter 2 in Längsrichtung d.h. axialer Richtung verschiebbar.

Die jeweiligen Führungsfederhalter 31 können am Innenumfangsabschnitt der ersten Lagerschale 4 oder am distalen Rohrabschnitt 7A fest verankert sein. Beispielsweise können die Führungsfederhalter 31 am Innenumfangsabschnitt der ersten Lagerschale 4 oder am distalen Rohrabschnitt 7A angeklebt sein.

Im Ausführungsbeispiel sind die ein Paar bildenden Steuerdrähte 9 an der proximalen Seite verbunden. Die ein Paar bildenden Steuerdrähte 9 sind auch an der distalen Seite verbunden.

Alternativ können die Steuerdrähte 9 als vier völlig separate Steuerdrähte 9 angewendet werden.

Gemäss der Erfindung ist am Scheibenelement von Scheibe zu Scheibe der Überbrückungsabschnitt in Umfangsrichtung um ein Viertel des Gesamtumfangs (90 Grad) versetzt angeordnet.

### Bezugszeichenliste

- 1: Einführschlauch des Endoskops
- 2: Kontrollkörpergehäuse; Steuerkörperhalter
- 3: Führungsfeder der Zugseile
- 4: halbkugelförmiges Lager; erste Lagerschale
- 5: Ringelement
- 6: Joystickkopf; zweite Lagerschale
- 7: elastisches Stabelement; Steuerelement
- 8: Abdeckelement; Steuerelement
- 9: Steuerdraht; Zugseil
- 31: Führungsfederhalter
- 70: Scheibenelement
- 71: erste Scheibe
- 72: zweite Scheibe
- 73: dritte Scheibe
- 74: vierte Scheibe
- 75: fünfte Scheibe
- 76: Überbrückungsabschnitt
- 77: Überbrückungsabschnitt
- 78: Überbrückungsabschnitt
- 79: Überbrückungsabschnitt
- 90: Führungsöffnungen

## Patentansprüche

1. Endoskopsteuervorrichtung mit
einem Steuerkörperhalter (2),
einem joystickartigen Steuerelement (7, 8) zum Bewirken einer Auslenkbewegung und
zumindest einem Steuerdraht (9), der durch den Steuerkörperhalter (2) geführt ist und der die Auslenkbewegung des Steuerelements (7, 8) zu einem zu steuernden Element im Endoskop überträgt;
wobei das Steuerelement (7, 8) sich vom Steuerkörperhalter (2) in eine proximale Richtung erstreckt, und am Steuerelement (6, 7, 8) beabstandet vom Steuerkörperhalter (2) der zumindest eine Steuerdraht (9) befestigt ist,
wobei das Steuerelement (7, 8) ein elastisches Stabelement (7) aufweist, das bei Betätigen des Steuerelements (7, 8) zum Bewirken einer Auslenkbewegung abknickbar ist,
wobei das elastische Stabelement (7) zentrisch entlang seiner Längserstreckung am Außenumfang sich in radialer Richtung nach außen erstreckende parallele Scheiben (71-75) hat,
wobei die in Längsrichtung des Stabelementes (7) gesehen Zwischenräume zwischen den Scheiben (71-75) in der Anzahl der Steuerdrähte (9) vorgesehen sind,
wobei die Scheiben (71-75) am Außenumfangsrand Führungsöffnungen (90) zum Führen eines Steuerdrahtes (9) besitzen und die Führungsöffnungen (90) für den gleichen Steuerdraht (9) in Längsrichtung des Stabelementes (7) ausgerichtet sind,
wobei die Führungsöffnungen (90) für verschiedene Steuerdrähte (9) in Umfangsrichtung des Stabelementes (7) zueinander versetzt sind,
wobei die Anzahl der Steuerdrähte (9) vier beträgt, und
wobei die Scheiben (71-75) zwischen einem distalen Rohrabschnitt (7A) und einem proximalen Rohrabschnitt (7B) des elastischen Stabelementes (7) als einstückiges Scheibenelement eingebettet sind,
wobei
das Scheibenelement (71 - 75) als zylindrischer Körper aufgebaut ist, und einen größeren Außendurchmesser als der distale Rohrabschnitt (7A) und der proximale Rohrabschnitt (7B) hat
im Bereich des Außenumfangsrandes benachbarte Scheiben (71-75) durch Überbrückungsabschnitte (76-79) verbunden sind,
jeder Überbrückungsabschnitt (76-79) an benachbarten Scheiben (71-75) so ausgebildet ist, dass Umfangsrandabschnitte (76-79) der benachbarten Scheiben (71-75) an der zum Überbrückungsabschnitt (76-79) diametral entgegengesetzten Seite zueinander hin biegbar sind,
**dadurch gekennzeichnet, dass**
vier Überbrückungsabschnitte (76-79) von Scheibe zu Scheibe in Umfangsrichtung um einen Viertel des Gesamtumfangs versetzt angeordnet sind.

2. Endoskopsteuervorrichtung gemäß Anspruch 1, wobei
das Steuerelement (7, 8) einen Betätigungsabschnitt an der zum Steuerkörperhalter entgegengesetzten Seite hat,
wobei bei Betätigen des Steuerelements (7, 8) zum Spannen des zumindest einen Steuerdrahts (9) das Stabelement (7) geknickt wird.

3. Endoskopsteuervorrichtung gemäß Anspruch 2, wobei
der Betätigungsabschnitt ein Abdeckelement (8) aufweist, das das proximale Ende des Stabelementes (7) bedeckt, wobei zwischen Stabelement (7) und Abdeckelement (8) der zumindest eine Steuerdraht (3) geklemmt ist.

4. Endoskopsteuervorrichtung gemäß einem der Ansprüche 1 bis 3, wobei
das Stabelement (7) eine vordefinierte Sollknickstelle aufweist.

5. Endoskopsteuervorrichtung gemäß einem der Ansprüche 1 bis 4, wobei
die Endoskopsteuervorrichtung des Weiteren zwei ineinandergreifende halbkugelartige Lagerschalen (4, 6) aufweist, von denen eine erste Lagerschale (4) am Steuerkörperhalter (2) abgestützt ist und eine zweite Lagerschale (6) am proximalen Ende des Stabelementes (7) abgestützt ist, wobei die beiden Lagerschalen (4, 6) das elastische Stabelement (7) umgeben.

6. Endoskopsteuervorrichtung gemäß einem der Ansprüche 1 bis 5, wobei
das elastische Stabelement (7) relativ zum Steuerkörperhalter (2) verschiebbar ist.

7. Endoskopsteuervorrichtung gemäß einem der Ansprüche 1 bis 5, wobei
das elastische Stabelement (7) am Steuerkörperhalter (2) fixiert angeordnet ist.

8. Endoskopsteuervorrichtung gemäß einem der Ansprüche 1 bis 7, wobei
das elastische Stabelement (7) aus Kunststoff oder Metall hergestellt ist.

9. Endoskop mit einer Endoskopsteuervorrichtung gemäß einem der Ansprüche 1 bis 8.

## Claims

1. An endoscope control device comprising
a control body holder (2),
a joystick-like control element (7, 8) for effecting a deflection movement, and
at least one control wire (9) guided through the control body holder (2) and transmitting the deflection movement of the control element (7, 8) to an element to be controlled in the endoscope;
wherein the control element (7, 8) extends from the control body holder (2) in a proximal direction, and the at least one control wire (9) is fixed to the control element (6, 7, 8) in a manner spaced from the control body holder (2),
wherein the control element (7, 8) comprises an elastic rod element (7) which is bendable upon actuation of the control element (7, 8) so as to effect a deflection movement,
wherein centrally along the longitudinal extension on the outer circumference of the elastic rod element (7), the elastic rod element (7) has parallel discs (71-75) extending outwards in the radial direction,
wherein, seen in the longitudinal direction of the rod element (7), the spaces between the discs (71-75) are provided in the number of the control wires (9),
wherein the discs (71-75) have guide openings (90) on the outer circumferential edge for guiding a control wire (9), and the guide openings (90) for the same control wire (9) are aligned in the longitudinal direction of the rod element (7),
wherein the guide openings (90) for different control wires (9) are offset from each other in the circumferential direction of the rod element (7),
wherein the number of the control wires (9) is four,
wherein between a distal tube portion (7A) and a proximal tube portion (7B) of the rod element (7) the discs (71-75) are embedded as a disc member in one piece,
wherein the disc member (71-75) is made up as cylindrical body and has a larger outer diameter than the distal tube portion (7A) and the proximal tube portion (7B),
in the area of the outer circumferential edge, adjacent discs (71-75) are connected by bridging portions (76-79),
each bridging portion (76-79) is formed at adjacent discs (71-75) such that circumferential edge portions (76-79) of the adjacent discs (71-75) are bendable toward each other on the side diametrically opposite to the bridging portion (76-79),
**characterized in that**
from disc to disc in the circumferential direction, four bridging portions (76-79) are arranged offset by a quarter of the total circumference.

2. The endoscope control device according to claim 1, wherein
the control element (7, 8) has an actuating portion on the side opposite to the control body holder,
wherein, when the control element (7, 8) is actuated to tension the at least one control wire (9), the rod element (7) is bent.

3. The endoscope control device according to claim 1 or 2, wherein
the actuating portion comprises a cover element (8) which covers the proximal end of the rod element (7), with the at least one control wire (3) being clamped between the rod element (7) and the cover element (8).

4. The endoscope control device according to any one of claims 1 to 3, wherein
the rod element (7) comprises a predefined target bending position.

5. The endoscope control device according to any one of claims 1 to 4, wherein
the endoscope control device further comprises two interlocking hemispherical bearing shells (4, 6), a first bearing shell (4) of which is supported on the control body holder (2) and a second bearing shell (6) of which is supported on the proximal end of the rod element (7), the two bearing shells (4, 6) surrounding the elastic rod element (7).

6. The endoscope control device according to any one of claims 1 to 5, wherein
the elastic rod element (7) is displaceable relative to the control body holder (2).

7. The endoscope control device according to any one of claims 1 to 5, wherein
the elastic rod element (7) is fixedly arranged on the control body holder (2).

8. The endoscope control device according to any one of claims 1 to 7, wherein
the elastic rod element (7) is made of plastic or metal.

9. The endoscope comprising an endoscope control device according to any one of claims 1 to 8.

## Revendications

1. Dispositif de commande de l'endoscope comprenant
un support de corps de commande (2),
un élément de commande (7, 8) de type joystick pour provoquer un mouvement de déviation et
au moins un fil de commande (9) qui est guidé à travers le support du corps de commande (2) et qui transmet le mouvement de déviation de l'élément de commande (7, 8) à un élément à commander dans l'endoscope ;
l'élément de commande (7, 8) s'étendant depuis le support de corps de commande (2) dans une direction proximale, et l'au moins un fil de commande (9) étant fixé à l'élément de commande (6, 7, 8) à distance du support de corps de commande (2),
l'élément de commande (7, 8) présentant un élément de tige élastique (7) qui peut être plié lors de l'actionnement de l'élément de commande (7, 8) pour provoquer un mouvement de déviation,
dans lequel l'élément de tige élastique (7) a des disques parallèles (71-75) s'étendant vers l'extérieur dans la direction radiale au centre le long de son extension longitudinale sur la périphérie extérieure,
les espaces entre les disques (71-75), vus dans la direction longitudinale de l'élément de tige (7), étant prévus en nombre égal à celui des fils de commande (9),
les disques (71-75) possèdent sur le bord périphérique extérieur des ouvertures de guidage (90) pour guider un fil de commande (9) et les ouvertures de guidage (90) pour le même fil de commande (9) sont orientées dans la direction longitudinale de l'élément de tige (7),
les ouvertures de guidage (90) pour différents fils de commande (9) étant décalées les unes par rapport aux autres dans la direction circonférentielle de l'élément de tige (7),
le nombre de fils de commande (9) étant de quatre, et
dans lequel les disques (71-75) sont insérés entre une section tubulaire distale (7A) et une section tubulaire proximale (7B) de l'élément de tige élastique (7) sous la forme d'un élément de disque d'une seule pièce,
l'élément en forme de disque (71-75) étant construit comme un corps cylindrique, et ayant un diamètre extérieur plus grand que la section tubulaire distale (7A) et la section tubulaire proximale (7B),
dans la zone du bord périphérique extérieur, des disques adjacents (71-75) sont reliés par des sections de pontage (76-79),
chaque section de pontage (76-79) est formée sur des disques adjacents (71-75) de telle sorte que des sections de bord périphérique (76-79) des disques adjacents (71-75) peuvent être fléchies les unes vers les autres sur le côté diamétralement opposé à la section de pontage (76-79), **caractérisé en ce que**
quatre sections de pontage (76-79) sont décalées d'un disque à l'autre dans la direction circonférentielle d'un quart de la circonférence totale.

2. Dispositif de commande d'endoscope selon la revendication 1, dans lequel
l'élément de commande (7, 8) a une section d'actionnement sur le côté opposé au support du corps de commande,
dans lequel, lorsque l'élément de commande (7, 8) est actionné pour tendre ledit au moins un fil de commande (9), l'élément de tige (7) est plié.

3. Dispositif de commande d'endoscope selon la revendication 2, dans lequel
la partie d'actionnement comprend un élément de recouvrement (8) qui recouvre l'extrémité proximale de l'élément de tige (7), ledit au moins un fil de commande (3) étant serré entre l'élément de tige (7) et l'élément de recouvrement (8).

4. Dispositif de commande d'endoscope selon l'une quelconque des revendications 1 à 3, dans lequel
l'élément de tige (7) présente un point de pliage prédéfini.

5. Dispositif de commande d'endoscope selon l'une quelconque des revendications 1 à 4, dans lequel
le dispositif de commande d'endoscope comprend en outre deux coquilles de support hémisphériques (4, 6) s'emboîtant l'une dans l'autre, dont une première coquille de support (4) est supportée par le support de corps de commande (2) et une deuxième coquille de support (6) est supportée par l'extrémité proximale de l'élément de tige (7), les deux coquilles de support (4, 6) entourant l'élément de tige élastique (7).

6. Dispositif de commande d'endoscope selon l'une quelconque des revendications 1 à 5, dans lequel
l'élément de tige élastique (7) est déplaçable par rapport au support de corps de commande (2).

7. Dispositif de commande d'endoscope selon l'une quelconque des revendications 1 à 5, dans lequel
l'élément de tige élastique (7) est disposé de manière fixe sur le support de corps de commande (2).

8. Dispositif de commande d'endoscope selon l'une des revendications 1 à 7, dans lequel
l'élément de tige élastique (7) est fabriqué en matière plastique ou en métal.

9. Endoscope muni d'un dispositif de commande d'endoscope selon l'une quelconque des revendications 1 à 8.
